# EUROPÄISCHE PATENTSCHRIFT

(11) **EP 2 338 528 B1**
(45) Veröffentlichungstag und Bekanntmachung des Hinweises auf die Patenterteilung: **29.05.2013**
(21) Anmeldenummer: 09016006.0
(22) Anmeldetag: 24.12.2009
(51) Int. Cl.: A61L 15/26, A61L 15/60

(54) **Hydrogelmatrix mit erhöhter Absorptionskapazität für Flüssigkeiten**
Hydrogel matrix with increased absorption capacity for liquids
Matrice d'hydrogel dotée d'une capacité d'absorption élevée pour des liquides

(43) Veröffentlichungstag der Anmeldung: 29.06.2011
(73) Patentinhaber: Paul Hartmann AG, 89522 Heidenheim (DE)
(72) Erfinder: Junginger, Martin, 89568 Hermaringen (DE); Horny, Julie, 68540 Bollwiller (FR)

(56) Entgegenhaltungen:
- EP-A1- 0 528 091
- DE-A1-102005 027 656

## Beschreibung

Die Erfindung betrifft Wundauflagen insbesondere als Wundbehandlungsmittel in der Granulations- und Epithelisierungsphase. Diese Wundauflagen können insbesondere zur feuchten Wundbehandlung eingesetzt werden.

Die Heilung von Hautwunden beruht auf der Fähigkeit der Haut Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differentierungsphase zur Epithelisierung und Narbenbildung (Phase 3, Epithelisierungsphase).

Zur Unterstützung der einzelnen Wundheilungsphasen sind zahlreiche Vorschläge in der Literatur beschrieben. Insbesondere sind auch Wundauflagen mit Hydrogelen seit geraumer Zeit Gegenstand zahlreicher Artikel in der Fachliteratur als auch von Patentliteratur. So werden beispielsweise mit den europäischen Patenten EP 455 324 B1, EP 528 091 B1, EP 567 704 B1 oder EP 630 629 B1 transparente Hydrogel-Wundauflagen mit verschiedenen Konstruktionen beschrieben. Diese zum Teil vielschichtigen Wundauflagen umfassen ein wasserhaltiges oder dehydratisiertes Hydrogel als Wundkontaktschicht zur Behandlung von Brandwunden.
Die EP0426422B1 offenbart einen Wundverband mit einem Hydrogel auf der Basis eines Polyharnstoff/Polyurethan-Copolymers. Die beschriebenen Zusammensetzungen enthalten 15 bis 30 Gew. % eines mehrwertigen Alkohols. Das Verhältnis der reaktiven Gruppen von Isocyanat zu den Amingruppen beträgt bei den in der EP0426422B1 beschriebenen Hydrogel-Zusammensetzungen ca. 0,63 bis 1,40. Gemäß einer als bevorzugte bezeichneten Ausführungsform beträgt das Verhältnis 1,23. Eine Wundauflage, welche ein Hydrogel gemäß den in der EP0426422B1 beschriebenen Zusammensetzungen enthält, ist unter der Bezeichnung Hydrosorb^{®} (Paul Hartmann Ag, Deutschland) auf dem Markt. Die Wundauflage kann nach einem Zeitraum von 48 Stunden etwa die doppelte Menge des Eigengewichtes an Flüssigkeit absorbieren.

Weiterhin werden mit den internationalen Anmeldungen WO 02/ 38 097 A1, WO 02/ 47 761 A1, WO 03/ 011 352 A1, WO 03/ 086 255 A1, WO 2004/ 052 415 A1 oder EP 1 658 865 A1 Wundauflagen beschrieben, die ein Hydrogel und einen Polymerschaum umfassen.

In der Patentanmeldung des Anmelders, Anmeldenummer DE102008031183.9, welche Stand der Technik im Sinne von Artikel 54(3) EPÜ darstellt, ist eine mehrschichtige Wundauflage mit einer Wundkontaktschicht als erster Schicht und mindestens einer zweiten Schicht als absorbierende Schicht, die einen hydrophilen Polyurethanschaum umfasst, beschrieben. Die Wundkontaktschicht kann ein Hydrogel auf Basis eines Polyharnstoff/Polyurethan-Copolymers sein. Das Ausführungsbeispiel der DE102008031183.9 offenbart ein Hydrogel, welches 17,5 Gew. % Propylenglykol umfasst, wobei das Verhältnis der reaktiven Gruppen von Isocyanat zu den Amingruppen in dem beschriebenen Hydrogel 1,23 beträgt.

Bei der modernen Wundbehandlung besteht ein Bedürfnis nach Wundauflagen, welche die Heilung von Wunden beschleunigen und den natürlichen Wundheilungsprozess unterstützen. Geeignete Wundauflagen müssen dabei unterschiedliche erwünschte Eigenschaften in Kombination aufweisen. Neben einer sehr guten Haut- und Gewebeverträglichkeit soll die Wundauflage ein die Wundheilung förderndes feuchtes Milieu gewährleisten, wobei überschüssige Flüssigkeit absorbiert werden sollte. Weiterhin muss die Wundauflage über atraumatische Eigenschaften verfügen, d.h. die Wundauflage muss sich beim Verbandwechsel entfernen lassen, ohne frisch entstandenes Wundgewebe zu beschädigen. Günstig für die Wundheilung ist ein feuchtes Milieu, wobei ein alkalischer pH-Wert, insbesondere ein pH-Wert über 8, vermieden werden sollte. Gleichzeitig soll überschüssige Flüssigkeit aus dem Wundbereich abgeleitet und von der Wundauflage absorbiert werden. Überschüssige Flüssigkeit bzw. Wundexsudat kann ansonsten zur Mazeration des Wundrandes führen. In der Praxis wird deshalb von den Anwendern, wie Ärzte oder Pflegepersonal, häufig gewünscht, dass eine Wundauflage eine möglichst hohe Absorptionsfähigkeit aufweisen soll.

Ausgehend von den beschriebenen und dem Markt zur Verfügung stehenden Wundauflagen mit einer Polyharnstoff/Polyurethan-Copolymer Hydrogelmatrix ist es daher eine Aufgabe der vorliegenden Erfindung, eine verbesserte Wundauflage bereitzustellen. Insbesondere ist es eine Aufgabe der vorliegenden Erfindung eine verbesserte Wundauflage bereitzustellen, die zur Wundheilung in der Granulations- und/ oder Epithelisierungsphase eingesetzt werden kann. Darüber hinaus soll eine atraumatische Wundauflage bereitgestellt werden, die den pathologischen Zustand einer Wunde derart beeinflusst, dass ein normaler, natürlicher Wundheilungsverlauf stattfinden kann. Hierfür soll die Wundauflage eine ausreichende Menge an Feuchtigkeit der Wunde bereitstellen und gleichzeitig einen für die Wunde verträglichen pH-Wert in der Nähe des Neutralpunktes aufweisen. Insbesondere ist es wünschenswert, dass die Wundauflage ein hohes Absorptionsvermögen für Flüssigkeiten aufweist.

Zur Lösung dieser Aufgaben wird eine mehrschichtige Wundauflage gemäß Anspruch 1 vorgeschlagen. Demnach umfasst eine erfindungsgemäße mehrschichtige Wundauflage eine erste Schicht als absorbierende Schicht, die eine Hydrogelmatrix umfasst, und mindestens eine zweite Schicht, welche auf der von der Wunde abgewandten Seite auf der ersten Schicht aufgebracht ist, dadurch gekennzeichnet, dass die Hydrogelmatrix 54 bis 60 Gew. % Propylenglykol, ein Vorpolymer mit Isophorondiisocyanatendgruppen (im Folgenden als "Isocyanat" bezeichnet) und ein Diamin auf Polyethylenoxidbasis in einer Menge von insgesamt 38 bis 42 Gew. %, 0 bis 5 Gew. % eines anorganischen Chlorids, sowie Rest Wasser umfasst, wobei das Verhältnis der reaktiven Gruppen von Isocyanat zu den Amingruppen des Diamins 1,25 bis 1,35 betragen soll.

Bei der zweiten Schicht kann es sich um eine weitere absorbierende Schicht, um eine Flüssigkeits-Verteilungsschicht oder um eine Trägerschicht handeln.

Die vorgeschlagene mehrschichtige Wundauflage beruht auf der überraschenden Entdeckung, dass eine Hydrogelmatrix mit einer gegenüber den aus dem Stand der Technik bekannten Polyharnstoff/Polyurethan-Copolymer Hydrogelmatrices wesentlich erhöhten Absorptionsfähigkeit für Flüssigkeiten und gleichzeitig einem die Wundheilung fördernden pH-Wert erhalten werden kann, wenn das Hydrogel eine Zusammensetzung umfasst, welche
a) Propylenglykol als mehrwertigen Alkohol umfasst,
b) eine speziell ausgewählte Menge an Propylenglykol umfasst,
c) ein Vorpolymer mit Isophorondiisocyanatendgruppen und ein Diamin auf Polyethylenoxidbasis in einem speziell ausgewählten Anteil umfasst, wobei
d) ein bestimmtes Verhältnis ("NCO:NH2") der reaktiven Gruppen von Isocyanat ("NCO") zu den Amingruppen des Diamins ("NH2") vorhanden sein muss.
Weiterhin umfasst die Zusammensetzung 0 bis 5 Gew. % eines organischen Chlorids, sowie Rest Wasser.
Eine erfindungsgemäße Wundauflage mit einer derartigen Hydrogelmatrix kann zudem eine Klebrigkeit auf der Wundstelle und der umgebenden Haut aufweisen, wodurch das Anlegen des Verbandes erleichtert werden kann.

Eine Hydrogelmatrix, welche 54 bis 60 Gew. % Propylenglykol, ein Vorpolymer mit Isophorondiisocyanatendgruppen und ein Diamin auf Polyethylenoxidbasis in einer Menge von insgesamt 38 bis 42 Gew. %, 0 bis 5 Gew. %, vorzugsweise 0,5 bis 1,5 Gew. %, eines anorganischen Chlorids, sowie Rest Wasser umfasst, wobei das Verhältnis der reaktiven Gruppen von Isocyanat zu den Amingruppen des Diamins 1,25 bis 1,35 beträgt, weist einen die natürliche Wundheilung unterstützenden pH-Wert nahe des Neutralpunktes, beispielsweise von 7,48, auf. Weiterhin weist die Hydrogelmatrix eine hohe Absorptionsfähigkeit für Flüssigkeiten auf. So kann beispielsweise nach 48 Stunden die 5,2-fache Menge des Eigengewichtes an demineralisiertem Wasser absorbiert werden. Gleichfalls wurde gefunden, dass die Hydrogelmatrix, wenn sie in direktem Kontakt mit der Wunde steht, über eine Klebrigkeit an der Wundstelle verfügt. Eine Klebrigkeit (engl. Tack) an der Wundstelle ist erwünscht, da dies das Anlegen des Verbandes erleichtert. Zur Bewertung des Tack (Klebrigkeit auf der Wunde und der umgebenden Haut) wurde bei den umfangreichen im Zusammenhang mit der Erfindung durchgeführten Versuchen eine relative Skala von 1 bis 5 herangezogen. Die Bewertung erfolgte anhand des subjektiven Eindrucks einer Person anhand der Klebrigkeit verschiedener Produkte zwischen Zeigefinger und Gel. Auf dem Markt erhältliche Produkte wurden dabei von einer Person in 5 Kategorien eingeteilt. Ein Tack von 1 bedeutet dabei, dass die Wundauflage nicht auf gesunder Haut klebt, während ein Tack von 5 eine sehr hohe Klebrigkeit bedeutet. Ein Tack von 5 ist weniger erwünscht, da eine derart stark klebende Wundauflage beispielsweise beim Verbandswechsel zu einer Beschädigung der Wundränder führen kann. Die in der vorliegenden Erfindung offenbarte Hydrogel-Matrix kann eine geringe Klebrigkeit aufweisen, beispielsweise eine Klebrigkeit mit einem Wert von 2.

Um die erwünschten Eigenschaften der Hydrogelmatrix zu erhalten, ist es wesentlich, dass die Zusammensetzung der Hydrogelmatrix einerseits einen Propylenglykolanteil von 54 bis 60 Gew. % umfasst. Dies ist im Vergleich zu den im Stand der Technik üblicherweise beschriebenen Zusammensetzungen ein wesentlich höherer Gehalt an Propylenglykol. Anderseits soll die Zusammensetzung ein Vorpolymer mit Isophorondiisocyanatendgruppen und ein Diamin auf Polyethylenoxidbasis in einer Menge von insgesamt 38 bis 42 Gew. % umfassen, wobei ein auf den Bereich von 1,25 bis 1,35, eingestelltes Verhältnis der reaktiven Gruppen von Isocyanat zu den Amingruppen des Diamins vorliegen muss. Es wurde gefunden, dass bei einer Abweichung von der in der Erfindung offenbarten Zusammensetzung ein Hydrogel mit vergleichsweise ungünstigeren Eigenschaften erhalten wird, beispielsweise ein Hydrogel mit einem unerwünscht hohem pH-Wert von mehr als 8, ein Hydrogel mit einer unzureichenden Absorptionskapazität oder ein Hydrogel, welches nicht ausreichend aushärtet.
Besonders vorteilhafte Hydrogele können erhalten werden, wenn die Zusammensetzung 54 bis 60 Gew. %, vorzugsweise 56 bis 58 Gew. % Propylenglykol und ein Vorpolymer mit Isophorondüsocyanatendgruppen und ein Diamin auf Polyethylenoxidbasis in einer Menge von insgesamt 38 bis 42 Gew. %, vorzugsweise 39 bis 41 Gew. %, umfasst, wobei ein auf den Bereich von 1,27 bis 1,33, idealerweise auf 1,29 bis 1,31, eingestelltes Verhältnis der reaktiven Gruppen von Isocyanat zu den Amingruppen des Diamins vorliegt. Weiterhin umfasst die Zusammensetzung 0 bis 5 Gew. %, vorzugsweise 0,5 bis 1,5 Gew. %, idealerweise 0,9 Gew. % eines anorganischen Chlorids, wobei es sich vorzugsweise um Natriumchlorid handelt. Derartige Hydrogele weisen hervorragende Eigenschaften hinsichtlich Absorptionskapazität, pH-Wert und Stabilität auf. Die Hydrogele können zudem eine Klebrigkeit auf der Haut aufweisen, die bei einer Verwendung des Hydrogels als Wundkontaktschicht erwünscht ist.

Das Hydrogel kann erhalten werden, indem Wasser, Propylenglykol und NaCl vermischt werden und anschließend das aufgeschmolzene Amin unter Rühren hinzugegeben wird, bis eine homogene Lösung entstanden ist. Danach wird das Isocyanat zugegeben. Das Gel härtet sehr schnell aus, üblicherweise in wenigen Minuten, beispielsweise in 10 Minuten, und weist über ausreichende Stabilität auf. Das Aushärten kann, wenn dies erwünscht ist, durch ein Abkühlen der Zusammensetzung nach der Zugabe des lsocyanates verzögert werden. Das Verhältnis der reaktiven Gruppen von Isocyanat zu den Amingruppen des Diamins kann auf übliche Weise anhand der Molekulargewichte der verwendeten Ausgangsstoffe unter Berücksichtigung der Stoffqualität (Reinheit) berechnet und eingestellt werden. Um das Verhältnis der reaktiven Gruppen von Isocyanat zu den Amingruppen des Diamins möglichst genau einzustellen, ist es vorteilhaft, wenn der Amingehalt der Mischung aus Wasser, Propylenglykol, NaCl und Diamin exakt bestimmt wird, bevor das Isocyanat zugegeben wird. Zur Bestimmung des Amingehaltes können übliche analytische Methoden eingesetzt werden. Das Verhältnis der reaktiven Gruppen von Isocyanat zu den Amingruppen des Diamins kann dann unter Berücksichtigung des gemessenen Amingehaltes anhand der zugegebenen Menge an Isocyanat auf einen Wert von 1,25 bis 1,35, vorzugsweise 1,27 bis 1,33, idealerweise 1,29 bis 1,31, eingestellt werden. Besonders vorteilhafte Gele werden erhalten, wenn das Verhältnis der reaktiven Gruppen von Isocyanat zu den Amingruppen des Diamins auf 1,30 eingestellt wird.

Als besonders geeignet für die erfindungsgemäße mehrschichtige Wundauflage hat sich eine erste Schicht mit einer Hydrogelmatrix erwiesen, welche folgende Zusammensetzung umfasst:
57 Gew. % Propylenglykol
15,3 Gew. % eines Diamins auf Polyethylenoxidbasis, beispielsweise Jeffamine ED-2003
0,9 Gew. % NaCl
24,7 Gew. % eines lsocyanates, beispielsweise Aquapol PI-13000-3
Rest Wasser
Bei der Zusammensetzung beträgt das Verhältnis der reaktiven Gruppen von Isocyanat zu den Amingruppen des Diamins 1,30.

Eine erfindungsgemäße mehrschichtigen Wundauflage, welche die Hydrogel-Matrix und mindestens eine zweiten Schicht, welche auf der von der Wunde abgewandten Seite auf der ersten Schicht aufgebracht ist, umfasst, weist eine unerwartet hohe Absorptionskapazität für Flüssigkeiten auf.

Eine Ausführungsform der mehrschichtige Wundauflage umfassend eine Hydrogel-Matrix der vorgenannten Zusammensetzung, welche eine Dicke von 0,8 mm aufweist und einer zweiten Schicht, welche als Trägermaterial ausgebildet ist, kann nach 48 Stunden die ca. 5,2-fache Menge, bezogen auf das Eigengewicht, an Wasser absorbieren. Die Hydrogel-Zusammensetzung umfasst dabei 57 Gew. % Propylenglykol, ca. 24,7 Gew. % Aquapol Pl-13000-3 (ein Isocyanat), 15,3 Gew. % Jeffamine ED-2003 (ein Diamin auf Polyethylenoxidbasis), 0,9 Gew. % NaCl und Rest (ca. 3 Gew. %) Wasser. Das Verhältnis der reaktiven Gruppen von Isocyanat zu den Amingruppen des Diamins wird auf 1,30 eingestellt. Dabei kann das Verhältnis der reaktiven Gruppen von Isocyanat zu den Amingruppen des Diamins auf übliche Weise anhand der Molekulargewichte der verwendeten Ausgangsstoffe unter Berücksichtigung der Stoffqualität (Reinheit) berechnet und eingestellt werden. Um das Verhältnis möglichst exakt auf den Wert 1,30 einzustellen, ist es vorteilhaft den Amingehalt der Lösung aus Wasser, Propylenglykol, NaCl und Diamin zu bestimmen, und die Menge an Isocyanat entsprechend anzupassen. Dabei kann sich gegenüber der vorgenannten Menge an Isocyanat, beispielsweise 24,7 Gew. % Aquapol, eine geringfügige Abweichung ergeben. Eine Wundauflage mit einer derartigen Hydrogelmatrix zeigt einen die natürliche Wundheilung unterstützenden pH-Wert von 7,48. Weiterhin verfügt die Wundauflage über ein geringe Klebrigkeit auf der Haut, die mit einem Tack von 2 bewertet wurde.

Bei der mindestens einen zweiten Schicht der mehrschichtigen Wundauflage handelt es sich um eine weitere absorbierende Schicht, eine Flüssigkeits-Verteilungsschicht oder um eine Trägerschicht. Vorzugsweise handelt es sich bei der mindestens einen zweiten Schicht um eine Trägerschicht. Diese Trägerschicht kann aus verschiedenen Materialien bestehen. Üblicherweise werden in Wundauflagen textile Trägermaterialien, Nonwoven, Polymerfilme oder Polymerschäume eingesetzt. Diese Trägerschicht kann einen direkten Kontakt oder einen indirekten Kontakt zu der ersten Schicht, welche eine Hydrogelmatrix umfasst, aufweisen. Bei einem direkten Kontakt wird die Trägerschicht direkt auf die Hydrogelmatrix auflaminiert, wogegen bei einem indirekten Kontakt die Trägerschicht mittels eines Adhäsivs auf die Hydrogelmatrix aufgebracht ist. Dabei kann das Adhäsiv vollflächig oder lediglich in Teilbereichen zwischen der Trägerschicht und der absorbierenden Schicht aufgebracht sein.

Als Trägerschicht einer erfindungsgemäßen Wundauflage können insbesondere Polymerfilme oder Polymerschäume eingesetzt werden. Ganz besonders bevorzugt sind Polymerfilme oder Polymerschäume, die wasserundurchlässig sind und die eine hohe Wasserdampfdurchlässigkeit aufweisen. Hierzu sind besonders Filme oder Schäume geeignet, die aus Polyurethan, Polyester, Polyetherurethan, Polyesterurethan, Polyether-Polyamid-Copolymeren, Polyacrylat oder Polymethacrylat gefertigt werden. Insbesondere ist als Trägerschicht ein wasserundurchlässigen und wasserdampfdurchlässigen Polyurethanfilm oder einen wasserundurchlässigen und wasserdampfdurchlässigen Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 60 µm, insbesondere 20 bis 40 µm und ganz besonders bevorzugt von 25 bis 30 µm aufweisen. Die Wasserdampfdurchlässigkeit des Polymerfilms der Wundauflage weist vorzugsweise mindestens 300 g/ m²/ 24 Std., insbesondere mindestens 1000 g/ m2/ 24 Std. und ganz besonders bevorzugt mindestens 2000 g/ m²/ 24 Std. auf (gemessen nach DIN EN 13726, upright).
In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnden Fläche umgebenden Haut austreten kann. Als besonders bevorzugt sind solche Klebstoffe zu betrachten, die in einem dünnen Auftrag von 20 bis 35 g/ m² zusammen mit dem Film eine Wasserdampfdurchlässigkeit von mindestens 400 g/ m²/ 24 Std. und vorzugsweise von mindestens 1000 g/ m²/ 24 Std. (gemessen nach DIN EN 13726, upright) aufweisen.

Dabei kann es sich bei der zweiten Schicht einer erfindungsgemäßen Wundauflage um Trägerschicht mit einem Gitteraufdruck handeln, wobei die Trägerschicht in direktem Kontakt mit der ersten Schicht steht. Ein Gitteraufdruck kann dem behandelnden Arzt oder medizinischen Personal die Beurteilung einer Wunde erleichtern, beispielsweise die Bestimmung der Wundgröße.

Hierbei und im Folgenden soll im Zusammenhang mit der vorliegenden Erfindung-falls nichts anderes angegeben ist-jeder Gehalt eines Inhaltstoffes in Gewichtsprozent (Gew. %) bezogen auf das Gewicht der den Inhaltsstoff umfassenden Komponente verstanden sein.

Die Hydrogelmatrix soll insbesondere 54 bis 60 Gew. % des mehrwertigen Alkohols Propylenglykol umfassen. Insbesondere umfasst die Hydrogelmatrix 56 bis 58 Gew. % Propylenglykol und ganz besonders bevorzugt 57 Gew. % Propylenglykol. Dieser Alkohol ist hervorragend als Feuchtigkeitsspender geeignet und stellt somit für die die Wunde umgebende Haut eine pflegende Komponente dar.
Weiterhin soll die Hydrogelmatrix ein Vorpolymer mit isophorondiisocyanatendgruppen und ein Diamin auf Polyethylenoxidbasis in einer Menge von insgesamt 38 bis 42 Gew. %, vorzugsweise 39 bis 41 Gew. %, insbesondere 40 Gew. % umfassen.
Gleichzeitig ist es für die Erfindung wesentlich, dass das Verhältnis der reaktiven Gruppen von Isocyanat zu den Amingruppen des Diamins 1,25 bis 1,35 beträgt, da nur dann die erwünschte hohe Absorptionskapazität und der vorteilhafte pH-Wert der Hydrogelmatrix erhalten wird, ohne andere Eigenschaften, wie zum Beispiels die Stabilität des Hydrogels in unerwünschter Weise zu verändern. Vorzugsweise soll das Verhältnis der reaktiven Gruppen von Isocyanat zu den Amingruppen des Diamins 1,27 bis 1,33, insbesondere 1,29 bis 1,31, betragen. Besonders vorteilhaft ist ein Verhältnis der reaktiven Gruppen von Isocyanat zu den Amingruppen des Diamins von 1,30.

Weiterhin ist vorgesehen, dass die Hydrogelmatrix mindestens ein anorganisches Chlorid umfassen kann. Besonders geeignet sind in diesem Zusammenhang Natriumchlorid, Kaliumchlorid, Magnesiumchlorid, Kalziumchlorid oder Mischungen hiervon. Diese Salze simulieren in besonders guter Weise das Elektrolyt-Gemisch in einem von einer Wunde abgegebenen Wundserum. Damit stellt eine diese Salze umfassende Hydrogelmatrix durch ähnliche osmotische Verhältnisse zwischen Hydrogel und Wundexsudat einer Wunde ein besonders wundheilungsförderndes Klima zur Verfügung.

Hierbei kann vorgesehen sein, dass die Hydrogelmatrix 0 bis 5 Gew.-% mindestens eines anorganischen Chlorids umfasst. Insbesondere umfasst die Hydrogelmatrix 0,1 bis 3 Gew.-% eines anorganischen Chlorids und ganz besonders bevorzugt 0,5 bis 1,5 Gew.-% eines anorganischen Chlorids. Als sehr günstig hat sich dabei eine Hydrogelmatrix erwiesen, welche 0,9 Gew. % Natriumchlorid.enthält.

Eine Hydrogelmatrix umfassend 37 bis 43 Gew.-% Propylenglycol, ein Vorpolymer mit Isophorondiisocyanatendgruppen und ein Diamin auf Polyethylenoxidbasis in einer Menge von insgesamt 12 bis 16,5 Gew. %,0,5 bis 1,5 Gew.-% eines anorganischen Chlorids und Rest Wasser, wobei das Verhältnis der reaktiven Gruppen von Isocyanat zu den Amingruppen des Diamins 1,25 bis 1,35 beträgt, weist eine freie Absorption A₃ gemessen nach DIN EN 13726-1 (2002) von mindestens 1 g/ g und höchstens 10 g/ g auf. Die Hydrogelmatrix stellt ein nicht reizendes, flüssigkeitsabsorbierendes, vor Bakterien schützendes, polsterndes, hautartiges Medium bereit und ist somit besonders gut als Wundkontaktschicht geeignet.

Gemäß der vorliegenden Erfindung handelt es sich bei der ersten Schicht vorzugsweise um eine Wundkontaktschicht. Unter einer Wundkontaktschicht wird hierbei eine Schicht verstanden, welche sich im Gebrauch in direktem Kontakt mit der Wundoberfläche befinden kann. Die Hydrogelmatrix kann dabei unterschiedliche Funktionen in Bezug auf die zu behandelnde Wunde ausüben. Die Hydrogelmatrix als Wundkontaktschicht kann ein die Wundheilung unterstützendes Milieu bereitstellen. Dazu kann die Hydrogelmatrix der Wunde Feuchtigkeit bereitstellen und gleichzeitig überflüssige Flüssigkeit absorbieren. Ein die Wundheilung unterstützendes Milieu wird gleichfalls durch einen pH-Wert in der Nähe des Neutralpunktes, beispielsweise durch einen pH-Wert von 7,5, bereit gestellt. Weiterhin kann die Hydrogelmatrix die empfindliche Wundoberfläche vor Scherkräften schützen. Schließlich kann die Hydrogelmatrix der Wundauflage eine gewisse Klebrigkeit auf der Wundstelle verleihen. Dies erleichtert das Anlegen des Verbandes.

Die eine Hydrogelmatrix umfassende erste Schicht kann neben der Hydrogelmatrix mindestens eine weitere Schicht umfassen, beispielsweise einen Polymerfilm, eine Hydrokolloidmatrix, ein Polymernetz, ein Nonwoven oder ein Adhäsiv. Die mindestens eine weitere Schicht kann sich auf der im Gebrauch von der Wunde abgewandten Seite befinden. Gemäß dieser Ausführungsform der Erfindung wird die Wundkontaktschicht durch die mindestens eine weitere Schicht gebildet. Gemäß einer weiteren Ausführungsform der Erfindung kann sich die weitere Schicht zwischen der ersten Schicht und der zweiten Schicht befinden.

Gemäß einer vorteilhaften Ausbildung der Erfindung umfasst die Wundauflage eine erste Schicht als Wundkontaktschicht, welche eine Hydrogelmatrix umfasst, wobei die Hydrogelmatrix in einer Schichtdicke von 0,4 bis 5 mm, vorzugsweise 0,6 bis 2,5 mm, insbesondere 0,8 mm aufweist. Weiterhin haben sich Schichtdicken von 1,35 mm und von 2,0 mm als sehr praktikabel erwiesen, da mit einer Wundauflage, welche eine derartige Wundkontaktschicht umfasst, ein hohes Absorptionsvermögen für Flüssigkeiten erreicht wird. Hydrogelmatrices mit den vorgenannten Schichtdicken, insbesondere mit Schichtdicken zwischen 0,6 und 2,0 mm, können eine ausreichende Menge Flüssigkeit bzw. Wundexsudat aufnehmen, ohne zu sehr aufzutragen. Diese Schichtdicken können an jeder Stelle der Wundkontaktschicht gleich sein oder in verschiedenen Bereichen der Wundkontaktschicht verschiedene Werte annehmen.

Weiterhin bevorzugt kann die Hydrogelmatrix Strukturen umfassen, insbesondere linienförmige oder kreisförmige Vertiefungen, Erhebungen, Noppen oder netzartige Muster. Vorzugsweise befinden sich diese Strukturen auf der im Gebrauch der Wunde zugewandten Seite Oberfläche der Hydrogelmatrix. Vorzugsweise durchdringen die Strukturen die Hydrogelmatrix nicht vollständig. Vorzugsweise weisen die Strukturen eine Tiefe von mindestens 2 % und höchstens 50 %, insbesondere mindestens 5 % und höchstens 25 %, bezogen auf die Dicke der Hydrogelmatrix auf.

Üblicherweise wird auf die im Gebrauch zur Wunde hin zeigende Seite der Wundkontaktschicht eine geeignete Abdeckfolie bzw. eine Releasefolie aufgebracht, um die Wundkontaktschicht zu schützen. Dazu kann beispielsweise eine silikonisierte Polyethylenfolie oder eine silikonisierte Polyesterfolie eingesetzt werden. Die Releasefolie kann geeignete Hilfsmittel umfassen, welche ein Ablösen der Releasefolie erleichtern, beispielsweise durch Rückfaltung gebildete Griffleisten,.

Gemäß einem weiteren weiterführenden Gedanken der vorliegenden Erfindung, ist auch eine Wundauflage Gegenstand der vorliegenden Erfindung, die zwischen der Hydrogelmatrix und der zweiten Schicht eine Barriereschicht aufweist. Eine solche Barriereschicht kann beispielsweise einen Polymerfilm umfassen.

Gemäß einem weiterführenden Gedanken der vorliegenden Erfindung, ist auch eine mehrschichtige Wundauflage umfassend eine erste absorbierende Schicht als Wundkontaktschicht, die eine Hydrogelmatrix umfasst, eine zweite Schicht als Trägerschicht, die einen Polymerfilm umfasst und eine Verteilerschicht, ebenfalls Gegenstand der vorliegenden Erfindung. Insbesondere ist die Verteilerschicht mit der Wundkontaktschicht verbunden. Eine solche Wundauflage weist besonders vorteilhaft eine Verteilerschicht zwischen der absorbierenden Schicht und der Trägerschicht auf, die aus einem hydrophilen Polyurethanschaum besteht. Durch die Verteilerschicht wird erreicht, dass eine Verteilung der aufgenommen Wundflüssigkeiten über die gesamte Fläche der Wundauflage insbesondere oberhalb der absorbierenden Schicht ermöglicht wird, das heißt das die Aufnahme der Wundflüssigkeiten nicht nur in z-Richtung (von der Wunde weg in Richtung Trägerschicht), sondern auch in x-y-Richtung (über die Fläche der Wundauflage) erfolgt.

Gemäß einer weiteren vorteilhaften Ausführungsform umfasst die mehrschichtige Wundauflage eine erste Schicht, die eine Hydrogelmatrix als Wundkontaktschicht umfasst und eine zweite Schicht als Trägerschicht, wobei erste und zweite Schicht in direkten Kontakt zueinander stehen. Als Trägerschicht können bei dieser Ausführungsform gleichfalls alle oben erwähnten Materialien eingesetzt werden.

Nach einer weiteren vorteilhaften Ausbildung der Erfindung umfasst die mehrschichtige Wundauflage auf der im Gebrauch von der Wunde abgewandten Seite weiterhin eine Wunddokumentationsfolie, wie sie beispielsweise in der deutschen Patentanmeldung DE102005027656 beschrieben ist.

### Zeichnungen

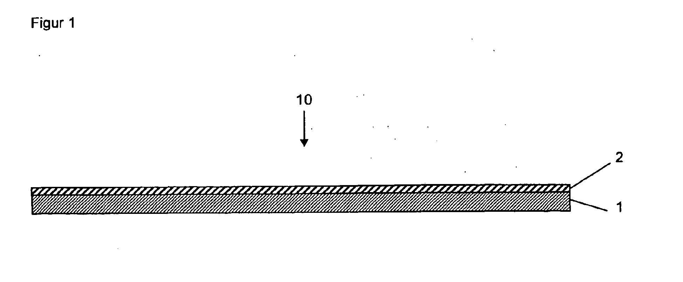

Mit Figur 1 ist eine Ausführungsform der erfindungsgemäßen Wundauflage (10) im Querschnitt gezeigt. Die Wundauflage (10) besteht aus einer ersten absorbierenden Schicht (1) und einer Trägerschicht (2). Die Trägerschicht (2) kann in direktem Kontakt mit der ersten Schicht (1) stehen oder durch eine Klebeschicht verbunden sein (hier nicht gezeigt). Auf der im Gebrauch von der Wunde abgewandten Seite der zweiten Schicht (2) kann weiterhin eine Applikationsfolie, welche vorzugsweise eine Abziehhilfe umfasst, aufgebracht sein (hier nicht gezeigt). Bei der ersten Schicht (1) handelt es sich um eine Wundkontaktschicht aus einer Hydrogelmatrix. Üblicherweise wird auf die Wundkontaktschicht (1), welche eine Hydrogelmatrix umfasst, eine Schutzschicht (Release-Folie), beispielsweise eine silikonisierte Polyurethan-Folie, aufgebracht (hier nicht gezeigt). Bei der zweiten Schicht (2) handelt es sich gemäß der in Figur 1 gezeigten Ausführungsform um eine Trägerschicht, beispielsweise aus Polyethylen oder Polyurethan. Die mehrschichtige Wundauflage (10) kann einen Kleberand aufweisen, d.h. als so genannter Inselverband gefertigt werden (hier nicht dargestellt). Ein Kleberand kann beispielsweise durch eine mit einem Klebeauftrag versehene Trägerfolie (2) ausgebildet werden, welche im gesamten Umfang der Wundauflage über die absorbierenden Schicht (1) hinausragt.

### Ausführungsbeispiel

### A) Herstellung des Hydrogels

Zur Herstellung des Hydrogels werden folgende wässrigen Lösungen und Komponenten (Komponente A, B, C) eingesetzt:

### Komponente A

| | |
|---|---|
| Propylenglycol USP30 (99,8 %) | Fa. Hedinger Aug GmbH; Stuttqart, Deutschland |
| Aqua purificata | Wasseraufbereitungsanlage |
| NaCl, reinst, USP | Fa. Hedinger Aug. GmbH; Stuttgart, Deutschland |

Zur Herstellung der Komponente A werden die Inhaltstoffe zusammengegeben und gerührt bis das Salz vollständig gelöst ist. Die Komponente A wird auf 2 °C abgekühlt.

### Komponente B

| | |
|---|---|
| Jeffamin ED-2003 | Fa. Huntsman; Everberg, Belgien |
| Aqua purificata | Wasseraufbereitungsanlage |

Zur Herstellung der wässrigen Komponente B wird das feste Jeffamin bei 56°C aufgeschmolzen und dem vorgelegten Wasser unter Rühren zugegeben. Die Komponente B wird auf Raumtemperatur abgekühlt.

### Komponente C

| | |
|---|---|
| Aquapol PI-13000-3 | Fa. Carpenter; Richmond, USA |

Die Komponente C wird auf Raumtemperatur gebracht.

Die vorbereiteten Komponenten A, B, und C werden miteinander homogen durch homogenisieren mittels eines rotierenden Mischsystems vermischt und in die bereitgestellten Formen möglichst blasenfrei gegossen.

| Versuch | Isocyanat ¹⁾ | Diamin ²⁾ | Polyharnstoff ³) | NCO /NH2 ⁴⁾ | Propylenglykol | Wasser | NaCl |
|---|---|---|---|---|---|---|---|
| 1.3 | 15,3 | 24,7 | 40 | 1,30 | 40 | 19,1 | 0,9 |
| 2.9 | 22,2 | 17,8 | 40 | 1,00 | 57 | 2,1 | 0,9 |
| 2.27 | 15,3 | 24,7 | 40 | 1,30 | 57 | 2,1 | 0,9 |
| 2.1.7 | 5,7 | 9,3 | 15 | 1,30 | 60 | 24,1 | 0,9 |

| | | | | | | | |
|---|---|---|---|---|---|---|---|
| Alle Angaben sind in Gew. % bezogen auf die Zusammensetzung. ¹⁾ Isocyanat Vorpolymer (Aquapol PI-13000-3), Anteil der reaktiven Gruppen (NCO) 3,223 % ²⁾ Diamin (Jeffamine ED-2003, Molekulargewicht 2.000), Amin-Gehalt 0,9554 mol/g ³⁾ Summe aus Isocyanat und Diamin ⁴⁾ Verhältnis der reaktiven Gruppen von Isocyanat zu den Amingruppen des Diamins | | | | | | | |

### B) Weitere verwendete Materialien

Als Trägerschicht wird ein 60 µm dicker, wasserundurchlässiger Polyurethanfilm (Fa. Exopack- Wrexham, United Kingdom) verwendet. Dieser Film ist mit einem PSA-Kleber (Pressure Sensitive Adhesive) auf Acrylatbasis beschichtet, wobei die Beschichtung eine Schichtdicke von 30 µm aufweist. Der Film weist eine Wasserdampfdurchlässigkeit MVTR (upright) von 1100 g/ m²/ 24 h (DIN EN 13726-1) auf.

Als Releasefolie wird ein eine 70µm dicke, einseitig silikonisierte LDPE-Folie, Typ SKV1002 (Fa. Deku Kunststofffabrik, Pommelsbrunn, D) verwendet. Die silikonisierte Seite ist zum Gel gerichtet.

### C) Herstellung der Wundauflagen

Die Wundauflagen (Muster) werden von Hand nach folgendem Ablauf gefertigt.
1. Zur Herstellung einer erfindungsgemäßen Wundauflage wird als erste Schicht eine Hydrogel-Zusammensetzung, wie vorstehend unter A) dargestellt, bereitgestellt. Dabei muss das Hydrogel nach dem Durchmischen und Homogenisieren sofort weiterverarbeitet werden. Zur Ausbildung der Hydrogelmatrix wird das Gel auf die Polyurethan-Folie gegossen.
2. Das Gel wird mit einer Rakel so verteilt, dass die Gelschicht eine Höhe von 0,8 mm aufweist.
3. Nach dem Polymerisieren, d.h. nach ca. 10 Minuten, wird das Hydrogel auf die vorbereitete Release-Folie gelegt (die silikonisierte Seite ist zum Gel gerichtet), so dass die zur Wunde gerichtete Seite geschützt ist.
4. Aus dem mehrlagigen Materialverbund werden Wundauflagen mit einer Kantenlänge von 10 x 10 cm ausgestanzt.

Die somit hergestellte Wundauflage weist den mit Figur 1 beschriebenen Aufbau auf, wobei mit Figur 1 kein Releaseliner gezeigt ist.

### D) Messmethoden

Die Absorptionsfähigkeit für demineralisiertes Wasser wird in Anlehnung an EN13726-1 bestimmt. Die Messung erfolgt bei 37°C. Es werden 2,5 x 2,5 cm große Probenstücke aus der Mitte der Hydrogelschicht geschnitten. Eine ggf. vorhandene Abdeckfolie wird abgezogen und entfernt. Die Proben werden in ein Becherglas eingewogen. Anschließend wird die 40-fache Menge an demineralisiertem Wasser zugeben. Das Becherglas wird mit einem Uhrglas abgedeckt. Nach 48h werden die Proben erneut gewogen. Die Wasseraufnahme wird in g Wasser pro g Gelstück berechnet (g / g).

Der pH-Wert einer Hydrogelmatrix wird im Zusammenhang mit der vorliegenden Erfindung durch Einlegen des Hydrogels in Wasser und Messung des pH-Wertes der Lösung bestimmt. Die Messung erfolgt bei Raumtemperatur (20°C). Es werden 2,5 x 2,5 cm große Probenstücke aus der Mitte der Hydrogelschicht geschnitten. Eine ggf. vorhandene Abdeckfolie wird abgezogen und entfernt. Die Proben werden in ein Becherglas eingewogen. Anschließend werden 12,5 ml demineralisiertes Wasser zugeben. Das Becherglas wird mit einem Uhrglas abgedeckt. Nach einem Zeitraum von 24 h wird die Probe aus der Lösung entfernt. Danach wird eine pH-Elektrode in die auf 20°C temperiert Lösung getaucht. Eine Ablesung des pH-Wertes erfolgt, sobald der angezeigte Zahlenwert stabil bleibt.

Die Klebrigkeit (Tack) der Wundauflage auf unversehrter Haut wurde abgeschätzt. Auf dem Markt erhältliche Produkte wurden dabei von einer Person in 5 Kategorien eingeteilt. Ein Tack von 1 bedeutet dabei, dass die Wundauflage nicht auf gesunder Haut klebt, während ein Tack von 5 eine sehr hohe Klebrigkeit bedeutet. Die Bewertung erfolgte anhand des subjektiven Eindrucks einer Person anhand der Klebrigkeit der hergestellten Wundauflagen zwischen Zeigefinder und Gel.

### E) Eigenschaften der Wundauflagen

| | pH ³) | Absorption ⁴⁾ | Tack |
|---|---|---|---|
| Wundauflage¹⁾ mit Hydrogel aus Versuch 1.3 | 7,51 | 3,33 g/g | 1 |
| Wundauflage ¹⁾ mit Hydrogel aus Versuch 2.9 | 8,99 | 9,56 g/g | 4 |
| Wundauflage ¹⁾mit Hydrogel aus Versuch 2.27 | 7,30 | 5,19 g/g | 2 |
| Hydrogel aus Versuch 2.1.7 | (Gel härtet nicht; Messung nicht möglich) | | |
| | | | |
| Hydrosorb Wundauflage ²⁾ | 8,06 | 2,00 | 1 |

| | | | |
|---|---|---|---|
| Die Hydrosorb Wundauflage (Paul Hartmann AG) enthält ein Hydrogel mit einer Schichtdicke von 2,0 mm. Das zur Herstellung von Hydrosorb verwendete Hydrogel umfasst 11,2 Gew. % Jeffamin ED-2003, 6,8 Gew. % Aquapol PI-13000-3, 18 Gew. % Propylenglykol und 63 Gew. % Wasser, 1 Gew. % NaCl. Das Verhältnis der reaktiven Gruppen von Isocyanat zu den Amingruppen des Diamins beträgt dabei 1,23. ¹⁾ Hydrogel Dicke 0,8 mm ²⁾ Hydrogel Dicke 2,0 mm ³⁾pH-Wert-Bestimmung, wie vorstehend unter D) beschrieben ⁴⁾ Die Absorption von demineralisiertem Wasser nach 48 h wurde in Anlehnung an EN13726-1 bestimmt wie unter D) dargestellt. | | | |

Die Wundauflage mit dem Hydrogel aus Versuch 1.3 weist einen die Wundheilung unterstützenden pH-Wert von 7,51 auf. Die Absorptionskapazität ist gegenüber dem aus dem Stand der Technik bekannten Hydrogel verbessert. Die Wundauflage weist keine Oberflächenklebrigkeit auf der Haut auf.

Die Wundauflage mit dem Hydrogel aus Versuch 2.9 weist einen für die Wundbehandlung nicht akzeptablen, sehr alkalischen pH-Wert von ca. 9 auf. Weiterhin zeigt die Wundauflage eine sehr hohe Absorptionsfähigkeit und einen Tack von 4, d.h. die Wundauflage haftet gut auf der Haut und lässt sich leicht entfernen. Der Tack liegt im erwünschten Bereich. Trotz hoher Absorptionsfähigkeit und vorteilhaften Tack ist die Wundauflage aufgrund des ungünstigen pH-Wertes nicht für eine die Wundheilung unterstützende Behandlung von Gewebedefekten geeignet.

Die Wundauflage mit dem Hydrogel aus Versuch 2.27 weist einen für die Wundbehandlung wünschenswerten pH-Wert von 7,30, d.h. nahe dem Neutralpunkt, auf. Weiterhin zeigt die Wundauflage eine hohe Absorptionsfähigkeit für Flüssigkeiten und einen Tack von 2, d.h. eine geringe Klebrigkeit auf der Haut. Die Wundauflage weist insgesamt eine sehr vorteilhafte Kombination von pH-Wert und Absorptionsfähigkeit auf.

Aus der in Versuch 2.1.7 getesteten Zusammensetzung konnte kein stabiles Hydrogel erhalten werden, da das Gel nicht aushärtete. Entsprechend war es nicht möglich eine mehrschichtige Wundauflage daraus herzustellen.

## Patentansprüche

1. Mehrschichtige Wundauflage umfassend
a) eine erste Schicht als absorbierende Schicht, die eine Hydrogelmatrix umfasst, und
b) mindestens eine zweiten Schicht, welche auf der von der Wunde abgewandten Seite auf der ersten Schicht aufgebracht ist,
**dadurch gekennzeichnet, dass** die Hydrogelmatrix 54 bis 60 Gew. % Propylenglykol, ein Vorpolymer mit Isophorondiisocyanatendgruppen und ein Diamin auf Polyethylenoxidbasis in einer Menge von insgesamt 38 bis 42 Gew. %, 0 bis 5 Gew. % eines anorganischen Chlorids, sowie Rest Wasser umfasst, wobei das Verhältnis der reaktiven Gruppen von Isocyanat zu den Amingruppen des Diamins 1,25 bis 1,35 betragen soll.

2. Wundauflage nach Anspruch1, wobei die Hydrogelmatrix 56 bis 58 Gew. % Propylenglykol und ein Vorpolymer mit Isophorondiisocyanatendgruppen und ein Diamin auf Polyethylenoxidbasis in einer Menge von insgesamt 39 bis 41 Gew. % umfasst.

3. Wundauflage nach mindestens einem der vorangehenden Ansprüche, wobei die Hydrogelmatrix 57 Gew. % Propylenglykol, 24,7 Gew. % eines Vorpolymers mit Isophorondiisocyanatendgruppen, 15,3 Gew. % eines Diamins auf Polyethylenoxidbasis, 0,9 Gew. % Natriumchlorid und Rest Wasser umfasst.

4. Wundauflage nach mindestens einem der vorangehenden Ansprüche, wobei es sich bei der zweiten Schicht um eine Trägerschicht handelt.

5. Wundauflage nach mindestens einem der vorangehenden Ansprüche, wobei es sich bei der zweiten Schicht um Filme oder Schäume aus Polyurethan, Polyetherurethan, Polyesterurethan, Polyether-Polyamid-Copolymeren, Polyacrylat, Polymethacrylat oder Mischungen daraus handelt.

6. Wundauflage nach mindestens einem der vorangehenden Ansprüche, wobei es sich bei der zweiten Schicht um eine adhesiv beschichtete Polymer-Folie mit einem Gitteraufdruck handelt, welche in direktem Kontakt mit der ersten Schicht steht.

7. Wundauflage nach Anspruch 6, wobei die Wundauflage eine weitere Polyurethan-Folie auf der im Gebrauch von der Wunde abgewandten Seite der Wundauflage umfasst.

8. Wundauflage nach mindestens einem der vorangehenden Ansprüche, wobei die Wundauflage weiterhin eine Release-Folie auf der im Gebrauch der Wunde zugewandten Seite der Wundauflage umfasst.

9. Wundauflage nach mindestens einem der vorangehenden Ansprüche, wobei die Wundauflage weiterhin eine Applikationsfolie auf der im Gebrauch von der Wunde abgewandten Seite der Wundauflage umfasst.

10. Wundauflage nach mindestens einem der vorangehenden Ansprüche, wobei die Wundauflage weiterhin eine Dokumentationsfolie umfasst.

## Claims

1. Multilayered wound dressing comprising
a) a first layer as absorbent layer comprising a hydrogel matrix, and
b) at least one second layer, applied atop the first layer on the wound-remote side,
**characterized in that** the hydrogel matrix comprises from 54 to 60 wt% of propylene glycol, altogether from 38 to 42 wt% of a prepolymer having isophorone diisocyanate end groups and of a diamine based on polyethylene oxide and from 0 to 5 wt% of an inorganic chloride, balance water, wherein the ratio for reactive groups of isocyanate to amine groups of diamine shall be in the range from 1.25 to 1.35.

2. Wound dressing according to Claim 1, wherein the hydrogel matrix comprises from 56 to 58 wt% of propylene glycol and altogether from 39 to 41 wt% of a prepolymer having isophorone diisocyanate end groups and of a diamine based on polyethylene oxide.

3. Wound dressing according to either or both of the preceding claims, wherein the hydrogel matrix comprises 57 wt% of propylene glycol, 24.7 wt% of a prepolymer having isophorone diisocyanate end groups, 15.3 wt% of a diamine based on polyethylene oxide, 0.9 wt% of sodium chloride, balance water.

4. Wound dressing according to at least one preceding claim, wherein the second layer is a backing layer.

5. Wound dressing according to at least one preceding claim, wherein the second layer comprises films or foams of polyurethane, polyetherurethane, polyesterurethane, polyether-polyamide copolymers, polyacrylate, polymethacrylate or mixtures thereof.

6. Wound dressing according to at least one preceding claim, wherein the second layer comprises an adhesive-coated polymer foil with a lattice imprint and in direct contact with the first layer.

7. Wound dressing according to Claim 6, wherein the wound dressing comprises a further polyurethane foil on that side of the wound dressing which in use is remote from the wound.

8. Wound dressing according to at least one preceding claim, wherein the wound dressing further comprises a release foil on that side of the wound dressing which in use faces the wound.

9. Wound dressing according to at least one preceding claim, wherein the wound dressing further comprises an application foil on that side of the wound dressing which in use is remote from the wound.

10. Wound dressing according to at least one preceding claim, wherein the wound dressing further comprises a documentation foil.

## Revendications

1. Pansement à plusieurs couches, comprenant
a) une première couche comme couche absorbante, qui comprend une matrice d'hydrogel, et
b) au moins une deuxième couche, qui est appliquée sur la première couche sur la face opposée à celle orientée vers la plaie,
**caractérisé en ce que** la matrice d'hydrogel comprend 54 à 60% en poids de propylèneglycol, un prépolymère présentant des groupes terminaux isophoronediisocyanate et une diamine à base de poly(oxyde d'éthylène) en une quantité totale de 38 à 42% en poids, 0 à 5% en poids d'un chlorure inorganique ainsi que, pour le reste, de l'eau, le rapport des groupes réactifs isocyanate aux groupes amine de la diamine valant 1,25 à 1,35.

2. Pansement selon la revendication 1, la matrice d'hydrogel comprenant 56 à 58% en poids de propylèneglycol et un prépolymère présentant des groupes terminaux isophoronediisocyanate et une diamine à base de poly(oxyde d'éthylène) en une quantité totale de 39 à 41% en poids.

3. Pansement selon au moins l'une quelconque des revendications précédentes, la matrice d'hydrogel comprenant 57% en poids de propylèneglycol, 24,7% en poids d'un prépolymère présentant des groupes terminaux isophoronediisocyanate, 15,3% en poids d'une diamine à base de poly(oxyde d'éthylène), 0,9% en poids de chlorure de sodium et pour le reste de l'eau.

4. Pansement selon au moins l'une quelconque des revendications précédentes, la deuxième couche étant une couche support.

5. Pansement selon au moins l'une quelconque des revendications précédentes, la deuxième couche étant des films ou des mousses de polyuréthane, de polyétheruréthane, de polyesteruréthane, de copolymères de polyéther-polyamide, de polyacrylate, de polyméthacrylate ou de mélanges de ceux-ci.

6. Pansement selon au moins l'une quelconque des revendications précédentes, la deuxième couche étant une feuille polymère revêtue de manière antiadhésive imprimée d'une grille, qui est en contact direct avec la première couche.

7. Pansement selon la revendication 6, le pansement présentant une autre feuille de polyuréthane sur la face du pansement opposée à la plaie lors de l'usage.

8. Pansement selon au moins l'une quelconque des revendications précédentes, le pansement présentant en outre une feuille antiadhésive sur la face du pansement orientée vers la plaie lors de l'usage.

9. Pansement selon au moins l'une quelconque des revendications précédentes, le pansement présentant en outre une feuille d'application sur la face du pansement opposée à la plaie lors de l'usage.

10. Pansement selon au moins l'une quelconque des revendications précédentes, le pansement présentant en outre une feuille de documentation.
